(19) Europäisches Patentamt
European Patent Office
Office européen des brevets

(11) Veröffentlichungsnummer: **0 000 775**
**A1**

(12) **EUROPÄISCHE PATENTANMELDUNG**

(21) Anmeldenummer: 78100587.1

(22) Anmeldetag: 03.08.78

(51) Int. Cl.²: C 08 L 21/00, A 61 L 15/00
D 06 M 15/28

(30) Priorität: 11.08.77 DE 2736205

(43) Veröffentlichungstag der Anmeldung:
21.02.79 Patentblatt 79/4

(84) Benannte Vertragsstaaten:
BE CH DE FR GB NL SE

(71) Anmelder: HOECHST AKTIENGESELLSCHAFT
KALLE, Patentabteilung Postfach 3540
D-6200 Wiesbaden 1(DE)

(72) Erfinder: Holst, Arno, Dr.
Am Hohen Stein 32
D-6200 Wiesbaden 1(DE)

(72) Erfinder: Schermann, Walter, Dr.
Blumenthalstrasse 20
D-6200 Wiesbaden 1(DE)

(72) Erfinder: Fischer, Wilhelm, Dr.
Hans-Sachs-Strasse 2
D-6780 Pirmasens(DE)

(54) **Wasserdampfaufnahmefähiges und wasserdampfdurchlässiges Flächengebilde aus Kautschuk und ein Verfahren zu seiner Herstellung.**

(57) Die Erfindung betrifft ein in der Wasserdampfaufnahmefähigkeit und -durchlässigkeit verbessertes Flächengebilde aus natürlichem oder synthetischem Kautschuk oder einem kautschukähnlichen Polymeren. Diese Flächengebilde, die beispielsweise als selbsttragende Folien oder Beschichtungen auf Unterlagen vorliegen, enthalten einen gleichmäßig eingearbeiteten Zusatz aus Teilchen aus mindestens einem quellfähigen, modifizierten Polymeren. Diese quellfähigen, modifizierten Polymeren sind insbesondere zu mindestens etwa 50 Gew.-% wasserunlöslich; zu ihnen zählen bevorzugt quellfähige, modifizierte Kohlenhydratderivate, wie mit Hilfe von Wärmeenergie, Strahlung oder durch eine zusätzliche chemische Verbindung vernetzte Stärke- oder Celluloseether.

Bei einem Verfahren zur Herstellung solcher Flächengebilde werden einer kautschukhaltigen Grundmasse die Teilchen aus mindestens einem quellfähigen, modifizierten Polymeren zugesetzt und darin gleichmäßig verteilt, und das Gemisch wird dann, gegebenenfalls unter Verschäumen, verstrichen oder anderweitig verformt und verfestigt.

Die erfindungsgemäßen Flächengebilde sind insbesondere für solche Anwendungsgebiete geeignet, bei denen unter physiologischen Bedingungen Körperflüssigkeiten, wie z.B. Schweiß, auftreten. Dazu zählen beispielsweise gummierte Textilien, Schuhobermaterialien, Schuheinlegesohlen, Teppichrückenmaterialien oder Hygieneartikel.

EP 0 000 775 A1

Croydon Printing Company Ltd.

H O E C H S T   A K T I E N G E S E L L S C H A F T
KALLE Niederlassung der Hoechst AG

Hoe 77/K 054 (K 2568)  1. August 1978
WLK-Dr.I.-db

# Wasserdampfaufnahmefähiges und wasserdampfdurchlässiges Flächengebilde aus Kautschuk und ein Verfahren zu seiner Herstellung

Die Erfindung betrifft ein in der Wasserdampfaufnahmefähigkeit und -durchlässigkeit verbessertes Flächengebilde aus natürlichem oder synthetischem Kautschuk oder einem kautschukähnlichen Polymeren und Verfahren zur Herstellung dieser Flächengebilde.

Flächengebilde aus Kautschuk werden auf verschiedenen technischen Gebieten angewandt, dazu zählen z. B. die Herstellung von Gummischuhen, Schwamm- und Zellgummi, gummierten Stoffen (z. B. Regenmäntel, Zelte, oder Teppichrückenbeschichtungen) oder von Tauchgummiwaren (z. B. Operationshandschuhe). Verfahren zur Herstellung solcher Flächengebilde, die meist als selbsttragende Folien oder als mehrschichtige (z. B. aus Deck- und Trägerschicht bestehende) Flächengebilde erzeugt werden, sind seit langem bekannt (siehe z. B. Ullmanns Encyklopädie der technischen Chemie, Verlag Urban & Schwarzenberg - München, 1957, Band 9, 3. Auflage, Stichwort "Kautschuk", insbesondere Seiten 351 ff, 365 ff und 375 ff oder S. Boström, Kautschuk-Handbuch, Verlag Berliner Union - Stuttgart, 1961, Band 4, 1. Auflage, insbesondere Seiten 172 ff und Seiten 263 ff). Werden diese Flächengebilde insbesondere unter physiologischen Bedingungen eingesetzt, d. h. beispielsweise als beschichtetes Textil oder als Schuh und in Form eines Schwamm(Schaum) -gummis für Schuh-Einlegesohlen, oder Polstermaterialien, so ist eine der entscheidenden Forderungen an das Material, daß es wasserdampfaufnahmefähig und zusätzlich möglichst auch noch wasserdampfdurchlässig ist, um beispielsweise auf dem Körper eine gute Tragebequemlichkeit zu erzeugen.

Da die genannten Flächengebilde häufig auf solchen Gebieten eingesetzt werden, bei denen eine wasserabstoßende Wirkung erzielt werden soll und sie dies erfolgreich bei einem relativ niedrigen Preis können, wird oftmals der damit einhergehende Nachteil der Nichtaufnahme oder Nichtdurchlässigkeit von Wasserdampf (z. B. aus Schweiß) in Kauf genommen. Es hat aber nicht an Versuchen gefehlt, diesen offensichtlichen Mangel zu beheben, insbesondere wurde versucht, die Porosität der Flächengebilde zu erhöhen. Durch diese Maßnahme, d. h. üblicherweise dem Einbringen von zusätzlichen Poren in das Flächengebilde aus Kautschuk, wird aber die Strukturfestigkeit der daraus erzeugten Materialien herabgesetzt und in einigen Fällen kann auch die an sich geforderte Wasserdichtigkeit der Materialien nicht beibehalten werden. Es wird bei diesen geschäumten Flächengebilden zwar eine gewisse Wasserdampfdurchlässigkeit beobachtet, aber eine nennenswerte Wasserdampfaufnahme ist nicht festzustellen.

Aus der DT-PS 910 960 ist ein Verfahren zur Herstellung poröser hochsaugfähiger Flächengebilde bekannt, das Produkte liefert, die neben den üblicherweise sich bildenden Hohlräumen noch künstlich erzeugte feine und feinste Poren enthalten und die deshalb saugfähig sein sollen. Die Erzeugung der zusätzlichen Feinporen erfolgt mit wasserlöslichen Stoffen, die in die Flächengebilde während deren Herstellung in feinverteilter Form eingearbeitet und aus den verfertigten Gebilden unter Zurücklassung feiner Poren wieder herausgelöst werden. Als Porenbildner dienen wasserlösliche organische Substanzen, wie Stärke, Eiweißstoff

oder eiweißartige Stoffe, Zucker, Tragant, Cellulosederivate und Kunstharze, oder solche Stoffe, die durch Einwirkung von Fermenten oder Enzymen wasserlöslich werden. Bei der Herstellung der Flächengebilde wird das Grundmaterial aus Vlies mit einem Imprägniermittel, das den Porenbildner enthält, behandelt; als Imprägniermittel können wäßrige Dispersionen oder Emulsionen von vulkanisierbaren Stoffen, wie Naturkautschuk, Kunstkautschuk oder Kunstharz verwendet werden.

In der DT-AS 12 04 186 wird ein Verfahren zur Herstellung eines fensterlederartigen, saugfähigen, porösen, beschichteten Gewebes beschrieben, bei dem ein Mischgewebe aus Baumwolle- und Zellwollfasern bis in den inneren Bereich der Gewebefäden aufgerauht, danach das Mischgewebe gereinigt und entschlichtet, anschließend die aufgerauhten Fasern des Mischgewebes mit einer Paste aus natürlichem oder synthetischem Kautschuklatex, einer die Viskosität erhöhenden Substanz und einem leicht-löslichen, zur Porenbildung geeigneten Salz ein- oder beidseitig dünn beschichtet und abschließend das Flächengebilde getrocknet und der Porenbildner ausgewaschen wird. Als die Viskosität erhöhende Substanzen werden Pflanzengummi, Tragant, Stärke, Dextrane und Gummi arabicum aufgeführt, als Porenbildner Natriumsulfat und -chlorid.

Das hydrophilierte Gebilde aus einem faser- und folienbildenden, wasserunlöslichen Polymeren gemäß der DT-OS 23 64 628 weist Teilchen aus modifiziertem Celluloseäther auf. Als Polymere sind die folgenden genannt: regenerierte

Cellulose (Cellulosehydrat), Celluloseacetat, Polyalkylen, Alkylcellulose, Polyacrylnitril, Polyamid und Polyester. Die modifizierten Celluloseäther sind solche, deren bloßer Verätherungsgrad zu wasserlöslichen Celluloseäthern führen würde und die derart modifiziert sind, daß sie mindestens zum größten Teil wasserunlöslich geworden, aber zur Wasseraufnahme befähigt geblieben sind. Das hydrophilierte Gebilde weist die Teilchen aus modifiziertem Celluloseäther in seiner polymeren Masse gleichmäßig verteilt oder eine mit den Teilchen bedeckte Oberfläche auf. Als technische Anwendungsgebiete für derartig hergestellte Folien werden die Verwendung als Ionenaustauscher oder als Dialyse- oder Osmose-Membranen angeführt.

Der Erfindung liegt die Aufgabe zugrunde, ein gegenüber dem Stand der Technik verbessertes wasserdampfaufnahmefähiges und -durchlässiges Flächengebilde auf der Basis von Kautschuk vorzuschlagen.

Die Erfindung geht aus von einem wasserdampfaufnahmefähigen und -durchlässigen Flächengebilde aus natürlichem oder synthetischem Kautschuk oder einem kautschukähnlichen Polymeren mit einem gleichmäßig eingearbeiteten Zusatz aus Polymeren-Teilchen. Das erfindungsgemäße Flächengebilde ist dadurch gekennzeichnet, daß es als Zusatz Teilchen aus mindestens einem quellfähigen, modifizierten Polymeren enthält. Unter quellfähigen Polymeren sind solche zu verstehen, die in wäßrigen Flüssigkeiten, insbesondere mit mehr als 50 Gew.-% Wassergehalt, oder durch anderweitig mit ihnen in Kontakt tretende Wassermoleküle (z. B. Wasserdampf) verursacht, quellen. Unter dem Begriff "gleichmäßig eingearbeitet" ist dabei eine statistische Verteilung zu verstehen. Das Polymere ist insbesondere zu mindestens etwa 50 Gew.-% wasserunlöslich.

HOECHST AKTIENGESELLSCHAFT
KALLE Niederlassung der Hoechst AG



In einer bevorzugten Ausführungsform enthält das Flächengebilde etwa 5 bis 30 Gew.-%, bezogen auf den Kautschukanteil, an Zusatz der Teilchen aus mindestens einem quellfähigen, modifizierten Polymeren. Die Teilchen haben zweckmäßig eine Größe von $\leq$ 250 µm, insbesondere von $\leq$ 150 µm und liegen im allgemeinen in pulvriger oder fasriger Form vor.

Als quellfähige, modifizierte Polymere sind für den Zusatz in den erfindungsgemäßen Materialien beispielsweise die folgenden geeignet:

Vernetztes Polyalkylenoxid nach der DT-OS 20 48 721; beim Verfahren zur Herstellung dieses Produkts werden wasserlösliche Polyalkylenoxide mit einer genügend starken ionisierenden Strahlung behandelt, um eine Vernetzung und ein Unlöslichwerden des Polymeren zu erreichen. Die Bestrahlung kann dabei mit dem Polyalkylenoxid in festem Zustand oder in Lösung erfolgen.

Das absorbierende, vernetzte, carboxylgruppenhaltige Mischpolymerisat nach der DT-OS 25 07 011 aus einer $\alpha$, β-ungesättigten Säure und einem Acetal der allgemeinen Formel

$$(CH_2 = CH-CH_2-O)_2 - CH - (CH_2)_n - CH - (O-CH_2-CH = CH_2)_2$$

wobei n = 0, 1 oder 2 ist. Als $\alpha$, β-ungesättigte Säure sind dabei insbesondere Acrylsäure, Methacrylsäure, Itaconsäure, $\alpha$-Phenylacrylsäure oder $\alpha$-Benzylacrylsäure geeignet; bei der Herstellung des Mischpolymerisats werden zweckmäßig auf 85 % bis 99,9 % einer der ungesättigten Säuren 0,1 % bis 15 % des Acetals verwendet.

Ein hydrokolloides Polymeres nach der US-PS 3.670.731 (= DT-OS 16 42 072), das durch Vernetzung wasserunlöslich gemacht wurde und dazu geeignet ist, Flüssigkeiten aufzunehmen und auch zurückzuhalten; genannt sind insbesondere bestimmte Polyacrylamide, Alkalimetallsalze von hydrolysierten Polyacrylamiden und Alkalimetallsalze von Polystyrolsulfonaten.

Ein in Wasser quellbares, vernetzes, unlösliches, physiologisch unschädliches Polymerisat nach der US-PS 3.669.103 (= DT-OS 16 17 998) aus der Gruppe der Poly-N-vinylpyrrolidone, Polyacrylamide, Polyacrylsäure und Polyglykole.

Nach dem Verfahren der DT-OS 25 41 035 hergestellte mindestens zum größten Teil wasserunlösliche, mit Wasser quellbare saugfähige Polymere, die derart hergestellt werden, daß man in homogener Phase Polyhydroxymethylen in wäßrig-alkalischer Lösung mit einer $\alpha$-Halogencarbonsäure veräthert und vor, während oder nach dem Veräthern mit einem gegenüber Polyhydroxymethylen in alkalischem Medium polyfunktionellen Vernetzungsmittel umsetzt.

Insbesondere sind die folgenden quellfähigen, modifizierten Kohlenhydratderivate im Rahmen der Erfindung verwendbar: Alkalimetallsalze der Carboxymethylcellulose, die wärmebehandelt werden und in Wasser quellfähig sind nach der US-PS 2.639.239; beim Verfahren zur Herstellung dieses Produkts wird die Löslichkeit eines wasserlöslichen Alkalimetallsalzes der Carboxymethylcellulose, die einen

D.S. (= Substitutionsgrad, d. h. Anzahl der substituierten Hydroxylgruppen an einer Anhydro-D-glucose-Einheit) von 0,5 bis etwa 1 aufweist, dadurch reduziert, daß man dieses trockene Salz in feinverteilter Form einer Temperatur von etwa 130° bis etwa 210° C aussetzt, wobei hochquellbare Gelteilchen erhalten werden.

Wasserunlösliche, Flüssigkeiten aufsaugende und zurückhaltende, wärmebehandelte Carboxyalkylcellulosen nach der US-PS 3.723.413 (= DT-OS 2 314 689); bei dem Verfahren zur Herstellung dieser Produkte geht man so vor, daß man

a) Cellulosematerialien mit carboxyalkylierenden Reaktionsteilnehmern behandelt und hierdurch wasserlösliche Carboxyalkylcellulose mit einem mittleren Substitutionsgrad von mehr als 0,35 Carboxyalkylresten pro Anhydroglucoseeinheit in der Cellulose, aber mit schlechten Eigenschaften in Bezug auf Aufsaugung und Zurückhaltung von Flüssigkeiten bildet,

b) einen solchen Teil der carboxyalkylierenden Reaktionsteilnehmer und während der Reaktion gebildeten Nebenprodukte entfernt, daß, bezogen auf das Gewicht der wasserlöslichen Carboxyalkylcellulose, wenigstens etwa 3 Gew.-% davon zurückbleiben, und

c) die Carboxyalkylcellulose in Gegenwart der verbliebenen carboxyalkylierenden Reaktionsteilnehmer und Nebenprodukte der Reaktion einer Wärmebehandlung

unterwirft und sie hierdurch wasserunlöslich macht und ihr ausgezeichnete Eigenschaften in Bezug auf Aufsaugung und Zurückhaltung von Flüssigkeiten verleiht.

Saugfähige Carboxymethylcellulosefasern, die zur Verwendung in Fasermaterialien für die Absorption und Retention wäßriger Lösungen geeignet und im wesentlichen wasserunlöslich sind nach der US-PS 3.589.364 (= DT-OS 1 912 740); derartige Fasern bestehen aus naßvernetzten Fasern von wasserlöslichen Salzen der Carboxymethylcellulose mit einem D.S. von etwa 0,4 bis 1,6 und weisen die ursprüngliche Faserstruktur auf. Als Vernetzungsmittel werden bevorzugt etwa 3 - 10 Gew.-% Epichlorhydrin eingesetzt.

Chemisch vernetzte, quellfähige Celluloseäther nach der US-PS 3.936.441 (= DT-OS 2 357 079); diese vernetzten Celluloseäther, insbesondere aus Carboxymethylcellulose, Carboxymethyl-hydroxyäthylcellulose, Hydroxyäthylcellulose oder Methyl-hydroxyäthylcellulose werden so hergestellt, daß die an sich wasserlöslichen Äther in alkalischem Reaktionsmedium mit einem Vernetzungsmittel umgesetzt werden, dessen funktionelle Gruppen

die Acrylamidogruppe $CH_2=CH-C(=\overline{O})-\overline{N}-$

die Chlor-azomethingruppe $-\overline{N}=C(-\overline{Cl})-$ oder

die Allyloxy-azomethingruppe $-\overline{N}=C(-)-\overline{O}-CH_2-CH=CH_2$

sind oder das Dichloressigsäure oder Phosphoroxychlorid ist.

Chemisch modifizierte, quellfähige Celluloseäther nach der US-PS 3 965 091 (= DT-OS 2 358 150); diese nicht durch Vernetzung modifizierten Celluloseäther werden so hergestellt, daß die an sich wasserlöslichen Äther in alkalischem Reaktionsmedium mit einer monofunktionell reagierenden Verbindung umgesetzt werden, die durch eine der beiden folgenden allgemeinen Formeln beschrieben wird:

$$\text{I} \qquad CH_2 = CH - CO - NH - \underset{\displaystyle R_2}{\underset{|}{CH}} - R_1 \qquad \text{oder}$$

$$\text{II} \qquad CH_2 = CH - SO_2 - NH_2 ,$$

wobei in Formel I: $R_1$ = die Hydroxyl-, eine Acylamino-oder eine veresterte Carbaminogruppe und $R_2$ = Wasserstoff oder die Carboxylgruppe bedeuten.

Chemisch vernetzte, quellfähige Celluloseäther nach der DT-OS 2 519 927; diese vernetzten Celluloseäther werden so hergestellt, daß die an sich wasserlöslichen Äther in alkalischem Reaktionsmedium mit Bisacrylamidoessigsäure als Vernetzer umgesetzt werden.

Frei fließende, durch Strahlung vernetzte, in Wasser quellbare hydrophile Kohlenhydrate nach der DT-AS 2 264 027; diese Produkte werden so hergestellt, daß man (mit

H O E C H S T   A K T I E N G E S E L L S C H A F T
KALLE Niederlassung der Hoechst AG



den folgenden Reaktionsstufen können auch bei bestimmten anderen Polymeren, wie Polyäthylenoxid oder Polyvinylalkohol, gleichartige Produkte erhalten werden):

a) mindestens ein wasserlösliches pulvriges polymeres Kohlenhydrat mit einer solchen Menge mindestens eines pulvrigen inerten Füllmittels, dessen Teilchen kleiner als die des Kohlenhydrates sind, so vermischt, daß ein wesentlicher Teil der Oberfläche des pulvrigen Kohlenhydrats bedeckt ist,

b) unter Fortsetzen des Mischens das Gemisch unter gründlichem Rühren mit einem feinverteilten Wasserspray in einer solchen Menge in Berührung bringt, bei der das Gemisch in einer frei fließenden Teilchenform erhalten bleibt, und

c) dann das erhaltene Gemisch bis zur Vernetzung des polymeren Kohlenhydrats einer ionisierenden Strahlung aussetzt.

Chemisch vernetzte oder anderweitig modifizierte, quellfähige Stärkeäther nach der DT-Anm. P 26 34 539.1; diese speziellen Stärkeäther werden so hergestellt, daß z. B. als Modifizierung eine Vernetzung mit einem Vernetzungsmittel durchgeführt wird, das folgende gegenüber Hydroxylgruppen reaktionsfähige funktionelle Gruppe trägt:

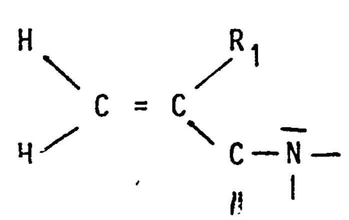

die Acrylamidogruppe, wobei $R_1$ = H oder $CH_3$ ist,

oder

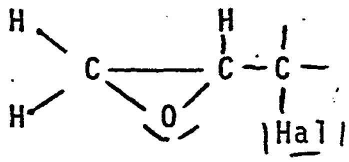

eine α-Halogen-epoxygruppe, wobei Hal = Cl oder Br ist

oder

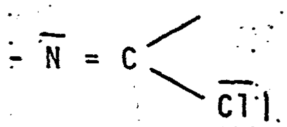

die Chlor-azomethingruppe

oder

$- \overline{N} = C - \overline{O} - CH_2 - CH = CH_2$ die Allyloxy-azomethingruppe

oder das Phosphoroxychlorid ist. Eine andere Art der Herstellung verläuft derart, daß die Modifizierung mit einer unter den genannten Bedingungen gegenüber den Hydroxylgruppen der Stärke oder des Stärkeäthers monofunktionell reaktionsfähigen Verbindung durchgeführt wird, die durch eine der folgenden allgemeinen Formeln beschrieben wird:

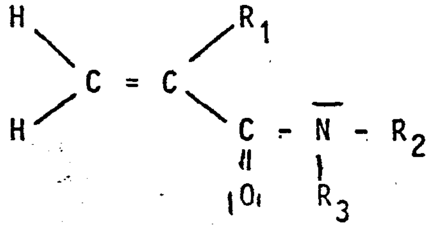

oder

H O E C H S T   A K T I E N G E S E L L S C H A F T
KALLE, Niederlassung der Hoechst AG



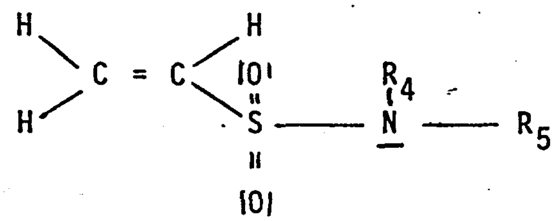

wobei $R_1 = CH_3$ oder H und $R_2$ = H und $R_3 = CH_3$, $CH_2$-OH, eine N-Methylen-acylamidogruppe mit 1 bis 3 C-Atomen, eine veresterte N-Methylen-carbamido- oder N-Carboxymethylencarbamidogruppe mit 2 bis 7 C-Atomen ist, oder $R_2$ und $R_3$ = $CH_3$ oder $CH_2$-OH sind und wobei $R_4$ und $R_5$ = H oder $R_4$ = H und $R_5 = CH_3$ oder $R_4$ und $R_5 = CH_3$ sind.

Alkalimetallsalze von Carboxymethylcellulose mit erhöhter Absorptions- und Retentionsfähigkeit nach der US-PS 3.678.031 (= DT-OS 21 51 973). Dabei werden zwar carboxylgruppenhaltige Verätherungsmittel eingesetzt, die einen normalerweise löslichen Celluloseäther zur Folge hätten, jedoch die Bedingungen der Reaktion so gewählt, daß Alkalimetallsalze von Carboxymethylcellulose mit einem D. S. von 0,4 - 1,2, einem wasserlöslichen Anteil von < 35 %, einem Wasserretentionswert (WRV) von etwa 1.000 bis 7.000 und einem Salzwasserretentionswert von etwa 400 bis etwa 2.500 entstehen.

Wasserunlösliche Carboxymethylcellulosen wie sie in den DT-PS 10 79 796 und DT-AS 11 51 474 verwendet werden, d. h. solche eines D. S. von 0,05 bis 0,3 und solche die im

wesentlichen wasserunlöslich sind und ebenfalls einen niedrigen D. S. aufweisen.

Wasserunlösliche, höher polymerisierte Carboxymethyl- oder Carboxyäthylcellulose mit einem wesentlichen Gehalt an freien Carboxylgruppen nach der GB-PS 725 887 (= DT-PS 10 37 076) die durch Erhitzen der wasserlöslichen, sauren Verbindungen auf 80° C bis 177° C wasserunlöslich gemacht werden.

Phosphorylierte Cellulosefasern nach der DT-OS 24 47 282, wie sie durch Umsetzung von Cellulose-Pulpe mit Harnstoff und Phosphorsäure unter Wärmeeinwirkung, einer anschließenden sauren Hydrolyse und einer abschließenden Oberführung in die Salzform erzeugt werden können.

Trockene, feste, mit Wasser quellbare, wasserunlösliche Absorptionsmittel nach der DT-OS 26 09 144, die aus einem ionischen Komplex von einem wasserunlöslichen anionischen Polyelektrolyten und einem Kation eines mindestens 3-wertigen Metalls bestehen; als Polyelektrolyte sind Polyacrylsäure, Stärke- oder Cellulosederivate geeignet.

Cellulosepfropfpolymerisate nach der DT-OS 25 16 380, die dadurch hergestellt werden, daß der Cellulose Seitenketten aus solchen Polymerisatresten aufgepfropft werden, die aus den ionischen und nichtionischen Polymerisatresten ausgewählt sind. Geeignet dazu sind beispielsweise: Polyacrylsäure, Natriumpolyacrylat, Polymethacrylsäure, Kaliumpolymethacrylat, Polyvinylalkoholsulfat, Polyphosphorsäure,

Polyvinylamin, Poly-(4-vinylpyridin), hydrolysiertes Polyacrylnitril, Polymethylmethacrylat, Polyvinylacetat, Polystyrol oder Polybutadien.

Granulierte, wasserunlösliche Alkalimetall-Carboxylat-Salze von Stärke-Acrylnitril-Pfropf-Copolymeren nach der US-PS 3.661.815, die durch Verseifung von Stärke-Acrylnitril-Pfropf-Copolymeren mit einer Base in einem wäßrig-alkalischen Medium hergestellt werden.

Modifiziertes Cellulosematerial mit verbessertem Rückhaltevermögen für sowohl Wasser als auch physiologische Flüssigkeiten nach der DT-OS 25 28 555, das durch Anpfropfen eines olefinisch ungesättigten, polymerisierbaren Monomeren mit hydrolysierbaren funktionellen Gruppen oder eines funktionelle Carboxylgruppen tragenden Monomeren auf ein faserförmiges Cellulosematerial und Hydrolysieren oder anderweitiges Behandeln des gepfropften Produktes mit Alkali hergestellt wird. Dabei wird das Produkt zunächst in den Zustand maximaler Quellung überführt, dann auf einen pH-Wert angesäuert, bei dem es den Zustand minimaler Quellung besitzt, anschließend unter keine Quellung bewirkenden Bedingungen in die Salzform überführt und abschließend getrocknet.

Modifiziertes Polysaccharid nach der DT-OS 26 47 420, hergestellt aus Polysaccharid, Acrylamid, einem anderen Vinylmonomeren und einem Divinylmonomeren unter radikalischen Reaktionsbedingungen.

Die Verfahren zur Herstellung von Flächengebilden aus Kautschuk sind bekannt. Das Flächengebilde kann dabei eine selbsttragende Folie sein oder durch Beschichten oder Imprägnieren einer Unterlage aus Natur- oder Synthesefasermaterial, nicht verwobenen Textilstoffen oder synthetischen Harzbahnen erzeugt werden; der Kautschukanteil kann dabei sowohl in massiver als auch in geschäumter Form vorliegen. Für das Beschichten oder Imprägnieren werden bevorzugt die folgenden Unterlagen verwendet:

Verwobene oder unverwobene Textilmaterialien aus einer oder mehreren Komponenten, z. B. aus Synthesefasern wie Polyamiden, Polyestern, Polyacrylnitril, PVC, Polyolefinen, Polyaminosäuren, sowie aus Glasfasern, regenerierten Fasern wie Viskosefasern, Acetatfasern und dgl., aus Naturfasern wie Baumwolle, Seide, Wolle, Leinen und Kollagen, erhalten durch Abreiben von Naturleder; oder blattartige Materialien, die aus einer oder mehreren Komponenten zusammengesetzt sind, z. B. aus synthetischen Harzen wie Polyamiden, Polyestern, Polyacrylnitril, PVC, Polyolefinen, Polyaminosäure, oder aus Naturleder, von dem die silbrige Oberfläche entfernt worden ist, oder aus Abfall-Leder erhaltenem Kollagen, Naturkautschuk und Synthesekautschuk.

Die Unterlagen können nach verschiedenen Verfahren beschichtet oder imprägniert werden, ebenso sind auch verschiedene Verfahren zur Herstellung selbsttragender Folien möglich. Das Grundmaterial zur Herstellung der erfindungsgemäßen Flächengebilde ist Kautschuk. Unter dem Oberbegriff Kautschuk sind beispielsweise die folgenden Unterbegriffe

zu verstehen: Latex aus Wild- oder Plantagenkautschuk, vorvulkanisierter Latex, Positex (Latex, dessen negative Ladung in eine positive umgewandelt wurde), Kautschuk-und Regenerat-Dispersionen, Rohkautschuktypen wie "Smoked Sheets" und "Crepe", synthetische Kautschuktypen wie Mischpolymerisate des Butadiens mit Styrol und des Butadiens mit Acrylnitril, Polymerisate und Mischpolymerisate des Chlorbutadiens, Polymerisate und umgewandelte Polymerisate aus Vinylverbindungen, Mischpolymerisate des Isobutylens mit Isopren, Blockpolymerisate des Butadiens, Thioplaste, Silicon-Kautschuk, regenerierter Kautschuk, Faktis und andere.

Die Verarbeitung des Kautschuks kann im allgemeinen wie folgt vorgenommen werden:

Die Verarbeitung von Latex erfolgt so, daß zunächst die Vulkanisationsagentien und sonstigen Hilfsprodukte wie Füllstoff, Alterungsschutzmittel, Weichmacher etc. in den Latex eingerührt werden, anschließend kann dann beispielsweise nach dem Tauchverfahren mit oder ohne Koagulationsmitteln, dem Schaumverfahren, durch elektrophoretische Abscheidung, dem Imprägnierverfahren oder dem Streichverfahren das gewünschte Flächengebilde erzeugt werden.

Die Verarbeitung von festem Kautschuk erfolgt so, daß zunächst die Rohkautschuk-Ballen zerkleinert werden, der Kautschuk plastifiziert und die zu verarbeitende Mischung mit üblichen Hilfsprodukten erzeugt wird, abschließend kann dann beispielsweise durch Kalandrieren, Friktionieren

und Tauchen oder Streichen von Gummilösungen das gewünschte Flächengebilde erzeugt werden, abschließend wird die Kautschukfolie oder die Kautschuk enthaltende Schicht vulkanisiert.

Bei der Durchführung der Beispiele wurde insbesondere nach den im folgenden beschriebenen Verfahren vorgegangen (Teile sind Gew.-Teile, %-Angaben sind in Gew.-% angegeben):

1 Eine Gummi-Textilbeschichtung wird erzeugt durch Auflösen von 30 Teilen der folgenden auf einer Walze erzeugten, vulkanisationsfähigen Mischung in 70 Teilen eines Benzin/Toluol-Gemisches aus 60 Teilen Benzin und 10 Teilen Toluol.

| | | |
|---|---|---|
| 60,0 | % | Smoked Sheets |
| 10,0 | " | Faktis |
| 20,0 | " | Ruß |
| 6,0 | " | Zinkoxid |
| 1,5 | " | Schwefel |
| 1,0 | " | Vulkanisationsbeschleuniger |
| 1,0 | " | Alterungsschutzmittel |
| 0,5 | " | Stearinsäure |

Vor dem Verstreichen oder einem andersartigen Verarbeiten werden der Lösung die Teilchen aus mindestens einem modifizierten, quellfähigen Polymeren zugesetzt und darin gleichmäßig verteilt, anschließend wird das Gemisch verstrichen oder andersartig verformt und abschließend während etwa 30 min bei etwa 120° C vulkanisiert.

2 Ein Schaumgummi wird erzeugt aus

| | | |
|---|---|---|
| 100,0 | Teilen | Crepe |
| 10,0 | " | Zinkoxid |
| 60,0 | " | Kreide als Füllstoff |
| 15,0 | " | Mineralöl |
| 2,0 | " | Paraffin |
| 3,0 | " | Stearinsäure |
| 1,0 | " | Formtrennmittel |
| 3,5 | " | Schwefel |
| 1,5 | " | Vulkanisationsbeschleuniger |
| 1,0 | " | Alterungsschutzmittel |
| 5,5 | " | Treibmittelpaste |

Vor dem Schäumen und Vulkanisieren werden diesem Gemisch die Teilchen aus mindestens einem modifizierten, quellfähigen Polymeren zugesetzt und darin gleichmäßig verteilt.

3 Latexschaumteile können ausgehend von Naturlatex oder Syntheselatex wie folgt hergestellt werden, wobei die Teilchen aus mindestens einem quellfähigen, modifizierten Polymeren vor dem Aufschlagen, dem Koagulieren und der Vulkanisation des Latexes, in etwa ihrer 4-fachen Wassermenge aufgeschlämmt, der Latexmischung zugesetzt und darin gleichmäßig verteilt werden.

3.1 Die Naturlatexmischung setzt sich zusammen aus:

| | | |
|---|---|---|
| 167,0 | Teilen | 60%igem Naturlatex |
| 3,5 | " | 20%iger wäßriger Na-stearat-Lösung |
| 3,0 | " | 10%iger wäßriger Dispergiermittel-Lösung |
| 2,5 | " | Schwefel |
| 1,6 | " | Vulkanisationsbeschleuniger |
| 7,8 | " | 5%iger wäßriger Emulgator-Lösung |
| 2,5 | " | Zinkoxid |
| 0,8 | " | Alterungsschutzmittel |
| 4,0 | " | Verdickungsmittel |
| 3,0 | " | 15%iger wäßriger Schaumstabilisator-Lösung |

Zum Koagulieren werden zu dem genannten Gemisch

20,0 Teile 10%iger wäßriger Ammoniumchlorid-Lösung

gegeben.

3.2 Die Syntheselatexmischung setzt sich zusammen aus:

| | | |
|---|---|---|
| 74,0 | Teilen | Buna-latex |
| 2,2 | " | K-oleat |
| 9,0 | " | Vulkanisierpaste |
| 5,0 | " | Methylcellulose |

Zum Koagulieren werden zu dem genannten Gemisch

2,0 - 4,0 Teile Silicofluorid gegeben.

Um die erfindungsgemäßen Flächengebilde herzustellen, werden also den zu verarbeitenden Grundmassen vor dem Verstreichen oder einer andersartigen Verformung die Teilchen aus mindestens einem modifizierten, quellfähigen Polymeren, bevorzugt in einem Anteil von 5 bis 30 Gew.-%, bezogen auf die fertige Folie bzw. die Schicht oder den Kautschukanteil in der Schicht, zugesetzt und darin gleichmäßig verteilt; das Gemisch wird dann verstrichen oder anderweitig verformt.

Die erfindungsgemäßen Flächengebilde weisen eine gute Fähigkeit zur Wasserdampfaufnahme und zur -durchlässigkeit auf, die über einen reinen Transporteffekt der eingearbeiteten Teilchen weit hinausgeht. Darüberhinaus sind die Flächengebilde auch dazu in der Lage, den aufgenommenen Wasserdampf unter bestimmten Bedingungen, beispielsweise Aufenthalt in einem andersartigen Klima, wieder abzugeben.

Da die genannten Eigenschaften des Flächengebildes nicht allein auf dem signifikant nachweisbaren Effekt durch den Zusatz der Teilchen aus mindestens einem quellfähigen, modifizierten Polymeren beruhen, sondern u. a. auch von der Stärke der Folie bzw. Beschichtung abhängig sind, wird diese zweckmäßigerweise in einer Stärke von etwa 0,05 bis zu etwa 0,5 mm hergestellt, insbesondere dann, wenn neben einer guten Wasserdampfaufnahmefähigkeit auch eine gute Wasserdampfdurchlässigkeit erzielt werden soll.

Die erfindungsgemäßen Flächengebilde mit den genannten Eigenschaften sind beispielsweise als selbsttragende Folie oder als Beschichtung auf einer Unterlage insbesondere als gummiertes Textil, Schuhobermaterial, Schuheinlegesohle, oder Teppichrückenmaterialien, also für solche Anwendungsgebiete geeignet, bei denen unter physiologischen Bedingungen Körperflüssigkeiten, wie z. B. Schweiß, auftreten. In geschäumter Form können sie auch als Saugstoff für Hygieneartikel, wie z. B. Tampons, eingesetzt werden, da sie als Schaum auch Wasser aufnehmen und zurückhalten können.

Unter den in der Beschreibung und den Beispielen zur Charakterisierung der erfindungsgemäßen Flächengebilde und den in ihnen vorhandenen quellfähigen Polymeren verwendeten Parametern ist folgendes zu verstehen:

WRV Wasserrückhaltevermögen des quellfähigen, modifizierten Polymeren in Gew.-%, gemessen gegen 2000-fache Erdbeschleunigung, bezogen auf seinen wasserunlöslichen Anteil; das WRV wird nach Eintauchen der Probe in Wasser bestimmt,

WUA wasserunlöslicher Anteil im quellfähigen modifizierten Polymeren,

DS Substitutionsgrad, Anzahl der substituierten Hydroxylgruppen an den Anhydro-D-glucose-Einheiten, von 0,0 bis 3,0

SV Saugvermögen des quellfähigen modifizierten Polymeren für 1%ige NaCl-Lösung in Gew.-%, bezogen auf sein Gesamtgewicht; das SV wird bestimmt nach Aufsaugen von 1%iger wäßriger NaCl-Lösung durch die Probe bis zur Sättigung.

$WDA_{DIN}$ Die Wasserdampfaufnahme wird über den Gewichtsverlust einer unter den Bedingungen nach DIN 53 304 (Ausgabe vom Mai 1968) bei 102° C ± 2° C bis zur Gewichtskonstanz getrockneten Probe, bezogen auf das ursprüngliche Gewicht, ermittelt. Dabei wird zunächst die Probe im Anlieferungszustand sofort nach der Entnahme aus einem wasserdampfdichten Behälter auf 0,001 g genau gewogen. Die Probestücke werden dann im Wärmeschrank hängend bei 102° C ± 2° C während 15 h getrocknet und nach dem Abkühlen auf Raumtemperatur ebenfalls auf 0,001 g genau gewogen. Um die Wasserdampfaufnahmefähigkeit von Proben unter verschiedenen Bedingungen ausprüfen zu können, werden die jeweiligen Proben in verschiedene Klimata gehängt und nach bestimmten Zeiten werden diese entnommen und dann deren Wasserdampfaufnahme in Gew.-%, bezogen auf ihr Anfangsgewicht zu Beginn der jeweiligen Messung, ermittelt.

$WDD_{PFI}$ Wasserdampfdurchlässigkeit (nach W. Fischer und W. Schmidt, "Das Leder", E. Roether-Verlag Darmstadt, 27, 87 ff (1976)). Innerhalb der

Apparatur herrscht eine Temperatur von 32° C, über der Probe ein Normklima - wenn nichts anderes angegeben ist - von 23° C / 50 % relative Feuchte, das von einem über dem Gerät angebrachten Ventilator durch einen leichten Luftstrom immer konstant gehalten wird. Die freie Prüffläche beträgt 10 cm². Auch innerhalb der Apparatur wird mit Hilfe eines Magnetrührers das Wasser von 32° C und die darüber befindliche mit Wasserdampf gesättigte Atmosphäre ständig in Bewegung gehalten. Die Bestimmung der WDD erfolgt durch die Bestimmung der Gewichtsabnahme des Prüfgefäßes mit der Probe. Die WDD wird in mg/cm² · x h (x meist 1, aber auch 8 oder 24) angegeben.

$WDA_{PFI}$ Wasserdampfaufnahme (siehe auch $WDD_{PFI}$). Die Wasserdampfaufnahme wird gleichzeitig mit der $WDD_{PFI}$-Messung durchgeführt, die Bestimmung erfolgt durch Ermittlung der Gewichtszunahme der Probe; wenn nichts anderes angegeben ist, ist die Probe zum Außenklima hin durchlässig, d. h. sie wird nicht abgedeckt.

In allen folgenden Beispielen wurden als repräsentative modifizierte, quellfähige Polymere mit Bisacrylamido-essigsäure chemisch-vernetzte Natrium-carboxymethylcellulosen mit den folgenden Parametern WUA $\geqq$ 70 %, WRV = 400 bis 700 %, SV = 800 bis 1.400 %, DS = 0,8 bis 1,1 und einer Teilchengröße von $\leqq$ 200 µm, mit einem Anteil von 90 Gew.-% von $\leqq$ 100 µm, verwendet.

Beispiele 1 bis 3 und Vergleichsbeispiel V1 (Tabelle I)

Es wird erfindungsgemäß ein gummibeschichtetes Textilgewebe aus Baumwolle mit einem Zusatz in der Schicht an modifiziertem, quellfähigem Polymeren in verschiedener Menge von 15 Gew.-% (Beispiel 1), 20 Gew.-% (Beispiel 2) und 25 Gew.-% (Beispiel 3), bezogen auf das Gewicht der Gummischicht, hergestellt und mit einem solchen ohne Zusatz (V1) bezüglich der Wasserdampfdurchlässigkeit und Wasserdampfaufnahme verglichen. Mit zunehmendem Anteil an modifiziertem, quellfähigem Polymeren nehmen diese Werte zu, es ergibt sich in den durchgeführten Fällen keine wesentliche Beeinflussung in den mechanischen Eigenschaften des beschichteten Gewebes. Die erfindungsgemäß beschichteten Gewebe mit Zusatz weisen im Vergleich mit den beschichteten Geweben ohne Zusatz eine deutliche Wasserdampfdurchlässigkeit und Wasserdampfaufnahme auf.

Beispiele 4 bis 6 und Vergleichsbeispiele V2 bis V4 (Tabelle II

Es wird erfindungsgemäß ein Schaumgummi aus einer Kautschukmasse (in verschiedener Dicke) mit einem Zusatz an modifiziertem, quellfähigem Polymeren in einem Anteil von 15 Gew.-%, bezogen auf das Gewicht des Schaumgummis, hergestellt und mit einem gleichdicken Schaumgummi ohne Zusatz bezüglich der Zunahme der Wasserdampfaufnahme nach steigender Einwirkungszeit des Wasserdampfs verglichen. Die Schaumgummis weisen folgenden Aufbau auf:

| | |
|---|---|
| Beispiel V2 u. Beispiel 4: | beide Außenflächen tragen eine Gummihaut |
| Beispiel V3 u. Beispiel 5: | eine Außenfläche zeigt einen offenen Schaum, eine Außenfläche trägt eine Gummihaut |
| Beispiel V4 u. Beispiel 6: | beide Außenflächen zeigen einen offenen Schaum |

Ein erfindungsgemäß hergestellter Schaumgummi mit Zusatz nimmt mehr Wasserdampf auf als ein gleichartiger Schaumgummi ohne Zusatz.

Beispiel 7 und Vergleichsbeispiel V5 (Tabelle III)

Es wird erfindungsgemäß ein Schaum aus Naturlatex mit einem Zusatz von 15 Gew.-% an modifiziertem, quellfähigem Polymeren, bezogen auf das Gewicht des fertigen Latexschaums, hergestellt (Beispiel 7) und mit einem solchen ohne Zusatz (V5) bezüglich der Wasserdampfaufnahme in verschiedenen Klimata und bei steigender Einwirkungszeit des Wasserdampfs verglichen. Ein erfindungsgemäß hergestellter Schaum aus Latex mit Zusatz nimmt wesentlich mehr Wasserdampf auf als ein gleichartiger Schaum ohne Zusatz.

Beispiele 8 bis 10 und Vergleichsbeispiel V 6 (Tabelle IV)

Es wird erfindungsgemäß ein Schaum aus Syntheselatex mit

einem Zusatz in verschiedener Menge von 10 Gew.-% (Beispiel 8), 20 Gew.-% (Beispiel 9) und 30 Gew.-% (Beispiel 10), bezogen auf das Gewicht des fertigen Latexschaums, hergestellt und mit einem solchen ohne Zusatz (V6) bezüglich der Wasserdampfaufnahme verglichen. Die Schäume werden einer Feuchte von 65 % relativer Feuchte (r.F.) bei 20° C ausgesetzt und anschließend verschiedenen Feuchten, und die jeweilige Feuchtezu- oder -abnahme wird nach bestimmter Zeitdauer gemessen. Mit zunehmender Menge an Zusatz steigt auch die Wasserdampfaufnahmefähigkeit des Schaumes, ein Schaum mit Zusatz nimmt wesentlich mehr Wasserdampf auf als ein gleichartiger Schaum ohne Zusatz.

Tabelle I

| Beispiel | Stärke der Probe (mm) | $WDD_{PFI}$ in mg/cm² · 8h | $WDD_{PFI}$ in mg/cm² · h | $WDA_{PFI}$ in mg/cm² · 8h | $WDA_{PFI}$ in Gew.-% |
|---|---|---|---|---|---|
| V1 | Gummischicht 0,2<br>Gewebeschicht 0,4 | 0,55 | 0,07 | 0,60 | 0,44 |
| 1 | " | 2,79 | 0,35 | 3,03 | 2,61 |
| 2 | " | 2,83 | 0,35 | 11,87 | 10,85 |
| 3 | " | 20,50 | 2,56 | 19,59 | 23,57 |

Tabelle II

| Beispiel | Stärke der Probe (mm) | Größe der Probe (mm²) | Zunahme der $WDA_{DIN}$ in Gew.-% *) nach | | | | | |
|---|---|---|---|---|---|---|---|---|
| | | | 1h | 2h | 4h | 8h | 12h | 24h |
| V2 | 25 | 50 · 100 | 0,03 | 0,05 | 0,07 | 0,12 | 0,16 | 0,25 |
| 4 | 25 | " | 0,11 | 0,17 | 0,27 | 0,45 | 0,61 | 0,95 |
| V3 | 5 | " | 0,10 | 0,14 | 0,23 | 0,37 | 0,50 | 0,82 |
| 5 | 5 | " | 0,46 | 0,68 | 0,99 | 1,50 | 1,90 | 3,01 |
| V4 | 20 | " | 0,04 | 0,05 | 0,08 | 0,13 | 0,16 | 0,26 |
| 6 | 20 | " | 0,44 | 0,62 | 1,00 | 1,54 | 2,01 | 3,13 |

*) Die Proben wurden einem Klima von 20° C / 65 % r. F. entnommen und bei 20° C / 95 % r. F. gelagert.

Tabelle III

| Beispiel | Stärke der Probe (mm) | Größe der Probe (mm²) | Meßdauer (h) | $WDA_{DIN}$ in Gew.-% im Klima von 20°C/20%r.F. | 20°C/35%r.F. | 20°C/65%r.F. | 20°C/95%r.F. |
|---|---|---|---|---|---|---|---|
| V5 | 5,5 | 50 · 100 | 4 | 0,01 | 0,03 | 0,04 | 0,45 |
| | | | 8 | 0,01 | 0,03 | 0,07 | 0,50 |
| | | | 12 | 0,01 | 0,04 | 0,08 | 0,88 |
| 7 | 5,5 | 50 · 100 | 4 | 0,80 | 1,20 | 1,92 | 7,22 |
| | | | 8 | 0,89 | 1,24 | 2,38 | 9,32 |
| | | | 12 | 0,94 | 1,28 | 2,41 | 10,83 |

Tabelle IV

| Beispiel | Stärke der Probe (mm) | Größe der Probe (mm²) | Meßdauer (h) | Zu- bzw. Abnahme (Gew.-%) der $WDA_{DIN}$ *) bei 20°C/20%r.F. | 20°C/35%r.F. | 20°C/95%r.F. |
|---|---|---|---|---|---|---|
| V6 | 5,5 | 50 · 100 | 4 | -0,06 | -0,03 | +0,39 |
| | | | 8 | -0,06 | -0,03 | +0,46 |
| | | | 24 | -0,06 | -0,03 | +0,80 |
| 8 | 5,5 | 50 · 100 | 4 | -0,48 | -0,13 | +3,30 |
| | | | 8 | -0,51 | -0,36 | +3,81 |
| | | | 24 | -0,53 | -0,36 | +6,35 |
| 9 | 5,5 | 50 · 100 | 4 | -1,31 | -0,96 | +6,51 |
| | | | 8 | -1,36 | -1,00 | +7,96 |
| | | | 24 | -1,40 | -1,03 | +13,51 |
| 10 | 5,5 | 50 · 100 | 4 | -1,98 | -1,47 | +7,84 |
| | | | 8 | -1,99 | -1,52 | +12,17 |
| | | | 24 | -2,12 | -1,57 | +18,21 |

*) Alle Werte sind auf die Anfangs-$WDA_{DIN}$ in einem Klima von 20° C / 65 % r. F. bezogen.

0000773

HOECHST AKTIENGESELLSCHAFT
KALLE Niederlassung der Hoechst AG

1. August 1978

Hoe 77/K 054 (K 2568) - 1 - WLK-Dr.I.-db

## Patentansprüche

1. Wasserdampfaufnahmefähiges und -durchlässiges Flächengebilde aus natürlichem oder synthetischem Kautschuk oder einem kautschukähnlichen Polymeren mit einem gleichmäßig eingearbeiteten Zusatz aus Polymeren-Teilchen, dadurch gekennzeichnet, daß es als Zusatz Teilchen aus mindestens einem quellfähigen, modifizierten Polymeren enthält.

2. Flächengebilde nach Anspruch 1, dadurch gekennzeichnet, daß das quellfähige, modifizierte Polymere zu mindestens etwa 50 Gew.-% wasserunlöslich ist.

3. Flächengebilde nach Anspruch 1 oder 2, dadurch gekennzeichnet, daß es als Zusatz Teilchen aus mindestens einem quellfähigen, modifizierten Kohlenhydratderivat enthält.

4. Flächengebilde nach einem der Ansprüche 1 bis 3, dadurch gekennzeichnet, daß es als Zusatz Teilchen aus mindestens einem quellfähigen, modifizierten Stärke- oder Celluloseäther enthält.

5. Flächengebilde nach einem der Ansprüche 1 bis 4, dadurch gekennzeichnet, daß der Stärke- oder Celluloseäther durch eine mit Hilfe von Wärmeenergie, Strahlung oder durch eine zusätzliche chemische Verbindung bewirkte Vernetzung modifiziert wurde.

H O E C H S T   A K T I E N G E S E L L S C H A F T
KALLE Niederlassung der Hoechst AG



6. Flächengebilde nach einem der Ansprüche 1 bis 5, dadurch gekennzeichnet, daß es etwa 5 bis 30 Gew.-% an Zusatz, bezogen auf den Kautschukanteil, enthält.

7. Verfahren zur Herstellung eines wasserdampfaufnahmefähigen und -durchlässigen Flächengebildes nach Anspruch 1, dadurch gekennzeichnet, daß einer kautschukhaltigen Grundmasse die Teilchen aus mindestens einem quellfähigen, modifizierten Polymeren zugesetzt und darin gleichmäßig verteilt werden, und das Gemisch dann, gegebenenfalls unter Verschäumen, verstrichen oder anderweitig verformt und verfestigt wird.

8. Verfahren nach Anspruch 7, dadurch gekennzeichnet, daß die Teilchen in einem Anteil von 5 bis 30 Gew.-%, bezogen auf den Kautschukanteil des fertigen Flächengebildes, zugesetzt werden.

0000775

Europäisches Patentamt

# EUROPÄISCHER RECHERCHENBERICHT

Nummer der Anmeldung
EP 78 10 0587

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US - A - 3 488 301 (T. KAWASHIMA)<br>* Spalte 1, Zeilen 45-49 *<br>-- | 1-8 |
| A | FR - A - 1 144 575 (O.W. BURKE)<br>* Zusammenfassung 1; Seite 6, rechts, 7. Absatz; Seite 9, rechts, 4. Absatz *<br>-- | |
| DA | DE - A - 2 364 628 (HOECHST)<br>* Patentanspruch; Seite 4, 1. Absatz *<br>-- | |
| PA | DE - A - 2 756 484 (HOECHST)<br>* Patentansprüche 1-6 *<br>-- | |
| PX<br>E | NL - A - 77 12778 (CPC INTERNATIONAL INC.)<br>& BE - A - 861 183<br>& FR - A - 2 372 205<br>& DE - A - 2 752 1233<br>* Patentanspruch 1; Seite 2, Zeilen 12-16 *<br>---- | 1-8 |

**KLASSIFIKATION DER ANMELDUNG (Int.Cl.²)**

C 08 L 21/00
A 61 L 15/00
D 06 M 15/28

**RECHERCHIERTE SACHGEBIETE (Int. Cl.²)**

C 08 L 7/00 -
C 08 L 21/02
A 61 L 15/00
D 06 M 15/28

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 06-11-1978 | VAN HUMBEECK |